Europäisches Patentamt

European Patent Office

Office européen des brevets

(11) Veröffentlichungsnummer: **0 083 773**
**B1**

(12) # EUROPÄISCHE PATENTSCHRIFT

(45) Veröffentlichungstag der Patentschrift:
**22.05.85**

(51) Int. Cl.⁴: **C 12 Q 1/56**, G 01 N 33/86,
**C 07 K 15/06**

(21) Anmeldenummer: **82111801.5**

(22) Anmeldetag: **20.12.82**

(54) Verfahren zur Herstellung eines Gewebsthromboplastinpräparates.

(30) Priorität: **21.12.81 DE 3150596**

(43) Veröffentlichungstag der Anmeldung:
**20.07.83 Patentblatt 83/29**

(45) Bekanntmachung des Hinweises auf die Patenterteilung:
**22.05.85 Patentblatt 85/21**

(84) Benannte Vertragsstaaten:
**AT BE CH DE FR GB IT LI LU NL SE**

(56) Entgegenhaltungen:
**DE - A - 2 316 430**
**DE - A - 2 356 493**
**DE - B - 1 189 763**
**US - A - 3 522 148**

(73) Patentinhaber: **Boehringer Mannheim GmbH,
Sandhoferstrasse 116, D-6800 Mannheim 31 (DE)**

(72) Erfinder: **Becker, Udo, Dr., rer.nat.,
Schmaedelstrasse 17, D-8000 München 60 (DE)**
Erfinder: **Schaich, Eugen, Dr., rer.nat., Deglergasse 6,
D-8120 Wellheim (DE)**
Erfinder: **Weigert, Manfred, Unteranger 1,
D-8132 Tutzing (DE)**

(74) Vertreter: **Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)**

## Beschreibung

Die Erfindung betrifft die Herstellung eines Gewebsthromboplastins (TP) aus Säugetiergewebe, welches eine Faktor-VII-Empfindlichkeit aufweist.

Unter einem TP versteht man nach DIN 58 910 einen Extrakt aus Säugetiergewebe, der in Anwesenheit von Kalziumionen die Gerinnungszeit von Blutplasma um ein Mehrfaches verkürzt.

Thromboplastine werden in der Diagnostik zur Erkennung von Gerinnungsstörungen verwendet. Der dabei angewandte Test wird als Quick-Test oder Prothombinzeittest bezeichnet. Man simuliert dabei die bei einer Gefässverletzung eintretenden Ereignisse, welche letztlich zur Blutgerinnung und zum Verschluss der Gefässwunde führen, indem man Gewebs-TP in Anwesenheit von Kalzium mit dem Blut oder Blutplasma des zu untersuchenden Patienten versetzt und die Zeit bis zur Bildung eines Gerinnsels misst. Das TP aktiviert dabei den Faktor VII, der seinerseits über die Faktoren X und V die Bildung von Thrombin aus Prothrombin (Faktor II) bewirkt. Thrombin spaltet das Fibrinogen zu unlöslichem Fibrin, welches am Wundverschluss beteiligt ist. Die Zeit, die nach Zugabe von Thromboplastin + Kalzium bis zur Bildung eines Gerinnsels vergeht, ist ein Mass für die Konzentration bzw. Aktivität der beteiligten Gerinnungsfaktoren.

Der Quick-Test gibt somit eine summarische Aussage über die Konzentrationsverhältnisse der beteiligten Gerinnungsenzyme (Faktoren). Dabei können bei gleichem Quick-Test durchaus unterschiedliche Verhältnisse in den Konzentrationen der einzelnen Faktoren II, V, VII und X vorliegen. Solche Verschiebungen in den Konzentrationsverhältnissen einzelner Gerinnungsfaktoren können, z. B. durch pathologische Veränderungen (Leberzirrhose, intravaskuläre Gerinnung, Vitaminmangel) oder auch durch Medikamente, z. B. Cumarinderivate zur oralen Antikoagulantientherapie, verursacht sein. Unterschiede werden dann erst sichtbar, wenn das gleiche Untersuchungsmaterial mit Thromboplastinen verschiedener Hersteller untersucht wird. Diese unterscheiden sich im allgemeinen stark in ihrer Empfindlichkeit gegenüber einzelnen Gerinnungsfaktoren, so dass dieselbe Patientenprobe mit TP verschiedener Hersteller ganz verschiedene Quick-Werte ergeben kann.

Man strebt daher an, die Vergleichbarkeit verschiedener Thromboplastine herbeizuführen. Ein Weg hierzu ist die Standardisierung in bezug auf eine definierte Faktorenempfindlichkeit. Es existieren zahlreiche Verfahren zur Herstellung von faktorempfindlichen TP. Während die Herstellung von Faktor-II-, -V- und -X-empfindlicher Präparate kein besonderes Problem mehr darstellt, bestehen noch Schwierigkeiten bei der Herstellung von Faktor-VII-empfindlichem Thromboplastin.

Es wird vermutet, dass eine geringe Faktorempfindlichkeit eines Thromboplastins dadurch bedingt ist, dass das Präparat noch Spuren von Gerinnungsfaktoren enthält, welche über den Blutgehalt des für die Thromboplastinextraktion verwendeten Gewebes eingebracht werden. Für diese Annahme spricht besonders ein in „Thrombosis Hemostasis" (Stuttgart) *1939*, 592-599 (1978), mitgeteilter Befund, wonach aus dem Gehirn von Hunden mit kongenitalem Mangel an Faktor VII ein besonders Faktor-VII-empfindliches Thromboplastin hergestellt werden kann.

Es wurden bereits Verfahren zur Herstellung speziell Faktor-VII-empfindlicher Thromboplastine beschrieben. Zum Beispiel soll der gewünschte Effekt gemäss DE-AS Nr. 2356493 dadurch erreicht werden, dass das Gewebe direkt mit einer Alkalilauge extrahiert wird. Eine Schädigung des Thromboplastins ist dabei in hohem Masse wahrscheinlich. Dies zeigt sich auch darin, dass bei Anwendung dieses Verfahrens eine grössere Anzahl von Hilfsstoffen zur Stabilisierung des erhaltenen Thromboplastins benötigt wird.

Ein weiteres Verfahren wird in „Biochem. Soc. Trans." *8*, 133 (1980), angegeben, nämlich die Adsorption von Faktor VII an Bariumsulfat. Dabei copräzipitiert jedoch nach eigenen Erfahrungen das Thromboplastin weitgehend mit dem Bariumsulfat.

Die Isolierung von Thromboplastin aus dem Gewebe von Tieren mit angeborenem Faktor-VII-Mangel kommt für die gewerbliche Anwendung schon aus Kostengründen und Gründen der Zugänglichkeit nicht in Frage.

Der Erfindung liegt daher die Aufgabe zugrunde, ein Verfahren zur Herstellung von Thromboplastin mit ausreichender und definierbarer Empfindlichkeit gegenüber Gerinnungsfaktor VII zu schaffen, welches die Nachteile der bekannten Verfahren nicht aufweist und es gestattet, das eingangs beschriebene Bedürfnis zu befriedigen.

Gelöst wird diese Aufgabe erfindungsgemäss durch ein Verfahren zur Herstellung eines Gewebsthromboplastinpräparates, welches gegen Gerinnungsfaktor VII empfindlich ist durch Herstellen von Acetontrockenpulver aus Säugetiergewebe und Extraktion des Pulvers mit Salzlösung, welches dadurch gekennzeichnet ist, dass man die Extraktion mit einer Salzlösung durchführt, welche 1 bis 20 mmol/l Ca-Ionen und gegebenenfalls ein oberflächenaktives Mittel enthält und den Extrakt gegebenenfalls trocknet.

Bevorzugt verwendet man erfindungsgemäss eine Salzlösung für die Extraktion, welche 5 bis 15 mmol/l Kalziumionen enthält. Als Kalziumsalz eignet sich dabei prinzipiell jedes ausreichend wasserlösliche Kalziumsalz. Bevorzugt verwendet man jedoch ein Kalziumsalz einer wasserlöslichen Carbonsäure, insbesondere der Ameisensäure oder Essigsäure.

Die Herstellung des Acetontrockenpulvers aus Säugetiergewebe erfolgt nach bekannten Verfahren. Sie ist beispielsweise beschrieben in „Human Blood Coagulation, Hemostasis and Thrombosis" (R. Biggs, Editor, Blackwell Scientific Publications, Oxford 1976, S. 663). Ausgehend von solchen acetongetrockneten Gewebspräparaten kann dann die erfindungsgemässe Extraktion des Thromboplastins erfolgen. Es ist jedoch zweckmässig, vor der eigentlichen Extraktion des Thromboplastins einen Waschschritt vorzuschalten, welcher zur Abtrennung noch im Gewebe erhaltener unerwünschter Verunreinigungen, wie z. B. Hämoglobin, dient. Wendet man den vorgeschalteten Waschschritt an, so erfolgt dies vorzugsweise mittels Pufferlösung eines pH-Werts von etwa 6,5 bis etwa 8. Beispielsweise kann diese Waschung mit 0,1 mol/l Natriumacetatpuffer pH 7,0 und Gewinnung des thromboplastinhaltigen Geweberückstandes durch physikalische Methoden wie Zentrifugieren erfolgen. Andere Waschflüssigkeiten sind jedoch ebenfalls geeignet. Ein derartiger vorgeschalteter Waschschritt führt allerdings zu einem gewissen Ausbeuteverlust durch Auswaschung von Thromboplastin.

Die eigentliche Extraktion mit einer kalziumionenhaltigen Salzlösung kann mit einer reinen Kalziumsalzlösung oder mit einer Lösung eines anderen Salzes, dem die erforderliche Menge an Kalziumionen zugesetzt wurde, erfolgen. Beispielsweise kann die Extraktion mit physiologischer Kochsalzlösung erfolgen, welcher die angegebene Menge an Kalziumionen in Form entsprechender Kalziumsalze zugesetzt wurde. Jedoch können auch andere Konzentrationen und andere Salze verwendet werden.

Ein Pufferzusatz ist in der Extraktionslösung nicht erforderlich, falls etwa neutrale Salze verwendet werden. Liegt jedoch der pH-Wert der verwendeten Salzlösung ausserhalb des Bereiches von etwa 5,5 bis 9, so wird zweckmässig ein Puffer zugesetzt, der einen annähernd neutralen pH-Wert sicherstellt.

Ein weiterer gegebenenfalls vorhandener Bestandteil der erfindungsgemäss verwendeten Extraktionslösung ist ein oberflächenaktives Mittel. Das oberflächenaktive Mittel ermöglicht eine Erhöhung der Ausbeute, ist jedoch für die Erzielung der Faktor-VII-Empfindlichkeit nicht notwendig. Geeignet sind sowohl kationenaktive, anionenaktive, als auch nichtionische oberflächenaktive Mittel. Ein typisches Beispiel für geeignete kationische Detergenzien ist Cetyltrimethylammoniumbromid, ein typisches Beispiel für anionische oberflächenaktive Mittel sind die Verbindungen der Gallensäuregruppe und ihre Salze wie beispielsweise Cholsäure, Desoxycholsäure usw. Ein Beispiel für geeignete nichtionische oberflächenaktive Mittel sind die Polyäthylenoxidäther und -ester mit hydrophoben Alkyl-, Aryl- und Aralkylresten.

Die jeweils geeignete Menge an oberflächenaktivem Mittel lässt sich durch einfache Vorversuche ausprobieren. Soweit es sich um anionische oberflächenaktive Mittel handelt, ist dafür Sorge zu tragen, dass die Löslichkeitsgrenze des gebildeten Kalziumsalzes nicht überschritten wird. Vorzugsweise setzt man 0,01 bis 0,5 Gew.-% oberflächenaktives Mittel der Extraktionslösung zu, jedoch können auch geringere und grössere Mengen zur Anwendung kommen.

Die Extraktion kann bei normaler oder erhöhter Temperatur durchgeführt werden. Bei erhöhter Temperatur lässt sich die Ausbeute erhöhen, jedoch sollte eine Obergrenze von etwa 45° C nicht überschritten werden, um eine Schädigung des extrahierten Thromboplastins zu vermeiden.

Eine Faktor-VII-Empfindlichkeit liegt dann vor, wenn bei Durchführung des Quick-Testes mit einem Faktor-VII-Mangelplasma eine wesentlich längere Gerinnungszeit gefunden wird als bei Durchführung des gleichen Tests unter Verwendung von Normalplasma. Unter Faktor-VII-Mangelplasma wird dabei ein Plasma verstanden, das von Personen mit kongenitalem Faktor-VII-Deffekt stammt.

Der erfindungsgemäss erzielte Effekt lässt sich z. B. zeigen, wenn man nach bekannten Verfahren hergestelltes Acetontrockenpulver zuerst mit Acetatpuffer pH 7,0 wäscht und dann mit 0,85%iger Kochsalzlösung, welche 0,05% Natriumdesoxycholat enthält, extrahiert. Das gewonnene TP zeigt im Quick-Test eine hohe thromboplastische Aktivität, z. B. gegenüber Normalplasma eine Gerinnungszeit von ca. 11 s. Verwendet man statt Normalplasma ein Faktor-VII-Mangelplasma, so findet man z. B. eine Gerinnungszeit von 18 bis 20 s. Das mit diesem kalziumfreien Extraktionsmittel extrahierte Thromboplastin hat daher nur eine geringe Faktor-VII-Empfindlichkeit.

Extrahiert man jedoch das Acetontrockengewebe mit einer entsprechenden Lösung, die zusätzlich 10 mmol/l Kalziumionen enthält, so zeigt der Thromboplastinüberstand nach Zentrifugieren im Quick-Test mit Normalplasma eine Gerinnungszeit von ca. 12 s, mit Faktor-VII-Mangelplasma dagegen eine Gerinnungszeit von 40 bis 60 s. Erfindungsgemäss erhält man also eine wesentlich höhere Empfindlichkeit gegenüber einem Faktor-VII-Mangelzustand als bei Anwendung eines bekannten Verfahrens mit Extraktion ohne Kalziumzusatz.

Charakteristischerweise wird dabei die Gerinnungszeit für Normalplasma bei erfindungsgemäss hergestelltem Thromboplastin etwas verlängert im Vergleich zu einem ohne Kalziumionen extrahierten Präparat. Dies fällt jedoch gegenüber dem starken Gewinn an Empfindlichkeit gegenüber Faktor VII nicht ins Gewicht. Drückt man beispielsweise die Faktorempfindlichkeit eines Thromboplastins als das Verhältnis der Gerinnungszeit eines Mangelplasmas zur Gerinnungszeit von Normalplasma aus, so zeigt ein nach dem vorstehenden Beispiel ohne Kalzium extrahiertes Thromboplastin ein Verhältnis von ca. 1,6, ein erfindungsgemäss gewonnenes hingegen ein solches von 4,0.

Ein Thromboplastin mit verbesserter Faktor-VII-Empfindlichkeit lässt sich zwar auch erhalten, wenn die untere Grenze von 1 mmol/l unterschritten wird, die erhaltenen Empfindlichkeitswerte liegen dann jedoch deutlich niedriger und sind zudem von Charge nicht einheitlich.

Ein weiterer Gegenstand der Erfindung ist ein Reagenz zur Kontrolle der Blutgerinnungsfähigkeit mit einem Gehalt an Thromboplastin und Kalziumsalz, welches dadurch gekennzeichnet ist, dass es ein nach dem hier beschriebenen Verfahren in Gegenwart von Kalziumionen hergestelltes Gewebsthromboplastinpräparat enthält. Vorzugsweise weist ein derartiges Präparat eine ganz definierte Faktor-VII-Empfindlichkeit auf. Dies lässt sich erreichen, indem man dem Präparat auch ein Thromboplastinpräparat zusetzt, welches gegen Faktor VII unempfindlich ist. Durch geeignete Auswahl der Mengen an Faktor-VII-empfindlichem und an Faktor-VII-unempfindlichem TP lässt sich jede gewünschte Faktor-VII-Empfindlichkeit einstellen. Dies lässt sich besonders einfach dadurch erreichen, dass man den Rückstand der erfindungsgemässen Extraktion des Acetontrockengewebes nochmals einer weiteren Extraktion ohne Kalziumionenzusatz unterwirft. Hierbei erhält man eine Faktor-VII-unempfindliche Thromboplastinfraktion. Durch Mischen unterschiedlicher Anteile der beiden Fraktionen lassen sich somit TP-Chargen in grossem Massstab herstellen, welche eine definierte und reproduzierbare Faktor-VII-Empfindlichkeit aufweisen. Hierdurch wird ein erheblicher Fortschritt bezüglich Standardisierung und Chargenkonstanz erreicht.

Ein besonderer Vorteil des erfindungsgemässen Verfahrens liegt in seiner Einfachheit und im Fehlen von Bedingungen, die eine Schädigung des Thromboplastins hervorrufen können.

Die folgenden Beispiele erläutern die Erfindung weiter.

*Beispiel 1*

a) 10 g Acetontrockenpulver aus Kaninchenhirn werden mit 10 g Supercel versetzt und mit 400 ml 0,1 mol Natriumacetatpuffer, pH 7,0, 30 min bei 37° C im Wasserbad inkubiert. Anschliessend wird 15 min lang bei 2500 g zentrifugiert. Der Niederschlag wird in 400 ml 0,85%iger Natriumchloridlösung, die 0,05% Natriumdesoxycholat enthält, suspendiert und 30 min lang bei 30° C im Wasserbad gerührt. Dann wird 10 min lang bei 2500 g zentrifugiert. Der Überstand enthält Faktor-VIIunempfindliches Thromboplastin. Der Niederschlag wird verworfen.

b) Die Extraktion des Thromboplastins erfolgt unter den gleichen Bedingungen wie in Beispiel 1a, jedoch mit einem Zusatz von 7,5 mmol/l Ca-Formiat. Man erhält ein Faktor-VII-empfindliches Thromboplastin.

*Beispiel 2*

Mit den nach Beispiel 1a und 1b hergestellten Thromboplastinextrakten wird ein Quick-Test durchgeführt. Da für die Reaktion Kalzium benötigt wird, muss der in Abwesenheit von Kalziumionen erhaltene Thromboplastinextrakt aus Beispiel 1a nachträglich mit Kalziumformiat auf 7,5 mmol/l aufgestockt werden. Als Normalplasma dient ein aus 10 gesunden Spendern gewonnenes Citratplasma, wie es nach DIN 58 910 zur Erstellung von Bezugskurven für den Quick-Test verwendet wird. Als Faktor-VII-Mangelplasma dient ein handelsübliches kongenitales Faktor-VII-Mangelplasma der Fa. Dade.

Je 0,1 ml Plasma werden mit je 0,2 ml der auf 37° C vorgewärmten Thromboplastinsuspension versetzt und die Zeit bis zum Gerinnungseintritt unter Verwendung eines Koagulometers nach Schnittger und Gross (Fa. Amelung, Lemgo, BRD) bestimmt. Man findet, dass die nach Beispiel 1a extrahierte Testthromboplastinsuspension mit Normalplasma nach 10,0 s, mit Faktor-VII-Mangelplasma nach 16,1 s, gerinnt (Verhältnis 1,6). Die unter Kalziumzusatz gemäss Beispiel 1b extrahierte Thromboplastinsuspension ergibt mit Normalplasma 12,3 s, mit Faktor-VII-Mangelplasma 45 s (Verhältnis 3,7) Gerinnungszeit.

*Beispiel 3*

Nach Beispiel 1a und 1b hergestellte Thromboplastinextrakte werden nach Ergänzung des Kalziumgehaltes des kalziumfreien Extraktes auf 7,5 mmol/l in verschiedenen Verhältnissen gemischt und die Gerinnungszeit entsprechend Beispiel 2 für Normal- und Faktor-VII-Mangelplasma gemessen. Die Ergebnisse zeigt Tabelle 1. Sie belegen, dass sich erfindungsgemäss Gemische mit definierter Faktor-VII-Empfindlichkeit herstellen lassen.

*Tabelle 1*

| Erhaltener Extrakt (Vol.-% ohne Ca) | Erhaltener Extrakt (Vol.-% mit Ca) | Gerinnungszeit Normalplasma (s) | Gerinnungszeit Faktor-VII- Mangelplasma (s) |
|---|---|---|---|
| 100 | 0 | 10,0 | 16,1 |
| 75 | 25 | 10,5 | 17,4 |
| 50 | 50 | 11,2 | 19,5 |
| 25 | 75 | 11,1 | 23,8 |
| 12,5 | 87,5 | 11,6 | 28,8 |
| 0 | 100 | 12,3 | 45,0 |

*Beispiel 4*

Zur Herstellung von Reagenz werden die Thromboplastinsuspensionen gemäss Beispiel 1a und 1b in solchen Anteilen gemischt, dass für Faktor-VII-Mangelplasma und Normalplasma die Gerinnungszeit ein Verhältnis von 3,1 ergibt. Sodann werden 2 Gew.-% Glycin sowie 0,01 Gew.-% Merthiolat hinzugefügt und die erhaltene Lösung lyophilisiert.

**Patentansprüche**

1. Verfahren zur Herstellung eines Gewebsthromboplastinpräparates, welches gegen Gerinnungsfaktor VII empfindlich ist, durch Herstellen von Acetontrockenpulver aus Säugetiergewebe und Extraktion des Pulvers mit Salzlösung, dadurch gekennzeichnet, dass man die Extraktion mit einer Salzlösung durchführt, welche 1 bis 20 mmol/l Ca-Ionen und gegebenenfalls ein oberflächenaktives Mittel enthält und den Extrakt gegebenenfalls trocknet.

2. Verfahren nach Anspruch 1, dadurch gekennzeichnet, dass man eine Salzlösung verwendet, welche 5 bis 15 mmol/l Kalziumionen enthält.

3. Verfahren nach einem der Ansprüche 1 oder 2, dadurch gekennzeichnet, dass man ein Kalziumsalz einer wasserlöslichen Carbonsäure verwendet.

4. Verfahren nach einem der vorhergehenden Ansprüche, dadurch gekennzeichnet, dass man das Acetontrockenpulver vor der Extraktion mit einer Pufferlösung von pH 6,5 bis 8 wäscht.

5. Verfahren nach einem der vorhergehenden Ansprüche, dadurch gekennzeichnet, dass man 0,1 bis 0,5% oberflächenaktives Mittel der Extraktionslösung zusetzt.

6. Reagenz zur Kontrolle der Blutgerinnungsfähigkeit mit einem Gehalt an Thromboplastin und Kalziumsalz, dadurch gekennzeichnet, dass es ein nach dem Verfahren der Ansprüche 1 bis 5 hergestelltes Gewebsthromboplastinpräparat enthält.

7. Reagenz nach Anspruch 6, dadurch gekennzeichnet, dass es ausserdem ein Thromboplastinpräparat, welches gegen Faktor VII unempfindlich ist, in solcher Menge enthält, dass eine vorherbestimmte Faktor-VII-Empfindlichkeit vorliegt.

## Claims

1. Process for the preparation of a tissue thromboplastin preparation, which is sensitive towards coagulation factor VII, by the preparation of acetone dry powder from mammalian tissue and extraction of the powder with salt solution, characterised in that one carries out the extraction with a salt solution which countains 1 to 20 mmol/l Ca ions and optionally a surface-active agent, and optionally dries the extract.

2. Process according to Claim 1, characterised in that one uses a salt solution which contains 5 to 15 mmol/l of calcium ions.

3. Process according to one of Claims 1 or 2, characterised in that one uses a calcium salt of a water-soluble carboxylic acid.

4. Process according to one of the preceding claims, characterised in that one washes the acetone dry powder before the extraction with a buffer solution of pH 6.5 to 8.

5. Process according to one of the preceding claims, characterised in that one adds 0.01 to 5% of surface-active agent to the extraction solution.

6. Reagent for the checking of the blood coagulation ability with a content of thromboplastin and calcium salt, characterised in that it contains a tissue thromboplastin preparation prepared according to the process of Claims 1 to 5.

7. Reagent according to Claim 6, characterised in that it also contains a thromboplastin preparation which is insensitive towards factor VII in such an amount that a predetermined factor VII sensitivity is present.

## Revendications

1. Procédé de préparation d'une composition de thromboplastine tissulaire, qui est sensible au facteur de coagulation VII, par préparation d'une poudre desséchée à l'acétone de tissu de mammifères et extraction de la poudre avec une solution saline, caractérisé en ce qu'on effectue l'extraction avec une solution saline qui contient 1 à 20 mmol/l d'ions Ca et le cas échéant un agent tensio-actif, et en ce qu'on sèche le cas échéant l'extrait.

2. Procédé suivant la revendication 1, caractérisé en ce qu'on utilise une solution saline qui contient 5 à 15 mmol/l d'ions calcium.

3. Procédé suivant l'une des revendications 1 ou 2, caractérisé en ce qu'on utilise un sel de calcium d'un acide carboxylique soluble dans l'eau.

4. Procédé suivant l'une des revendications précédentes, caractérisé en ce qu'on lave la poudre séchée à l'acétone avant l'extraction avec une solution tampon à pH 6,5 à 8.

5. Procédé suivant l'une des revendications précédentes, caractérisé en ce qu'on ajoute 0,01 à 0,5% d'un agent tensio-actif à la solution d'extraction.

6. Réactif pour le contrôle de l'aptitude à la coagulation du sang contenant de la thromboplastine et un sel de calcium, caractérisé en ce qu'il contient une préparation de thromboplastine tissulaire préparée suivant le procédé des revendications 1 à 5.

7. Réactif suivant la revendication 6, caractérisé en ce qu'il contient en outre une préparation de thromboplastine qui est insensible au facteur VII, dans une quantité telle qu'une sensibilité au facteur VII déterminée à l'avance est présente.